# EUROPÄISCHE PATENTSCHRIFT

(11) **EP 1 594 448 B1**
(45) Veröffentlichungstag und Bekanntmachung des Hinweises auf die Patenterteilung: **16.01.2008**
(21) Anmeldenummer: 04710012.8
(22) Anmeldetag: 11.02.2004
(51) Int. Cl.: A61Q 5/02, A61K 8/18, A61Q 5/12

(54) **HAARBEHANDLUNGSMITTEL MIT TENSIDMISCHUNGEN**
HAIR TREATMENT AGENTS WITH SURFACTANT MIXTURES
AGENTS DE TRAITEMENT CAPILLAIRE AVEC DES MELANGES DE TENSIOACTIFS

(30) Priorität: 19.02.2003 DE 10307115
(43) Veröffentlichungstag der Anmeldung: 16.11.2005
(73) Patentinhaber: Henkel Kommanditgesellschaft auf Aktien, 40589 Düsseldorf (DE)
(72) Erfinder: GODDINGER, Dieter, 25336 Klein Nordende (DE); HENTRICH, Dirk, 22457 Hamburg (DE)
(86) Internationale Anmeldenummer: PCT/EP2004/001264
(87) Internationale Veröffentlichungsnummer: WO 2004/073665

(56) Entgegenhaltungen:
- WO-A-97/25965
- DE-A- 19 741 162
- DE-A- 19 917 745
- DE-A- 19 964 155
- US-A- 5 422 031

## Beschreibung

Die Erfindung betrifft kosmetische Haarbehandlungsmittel auf der Basis einer Tensidmischung in Kombination mit speziellen Haarpflegestoffen sowie ein Verfahren zur gleichzeitigen Reinigung und Pflege der Haare mit diesen Mitteln.

Das menschliche Haar wird heutzutage mit einer Vielfalt kosmetischer Zubereitungen behandelt, die das Haar glänzend und gut kämmbar machen sollen. Gerade aber durch diese vielfältigen Behandlungsschritte, beispielsweise bei Bleichung, Färbung, Tönung, Verformung, aber auch bei Hitzeeinwirkung durch Föhnen oder mechanische Beanspruchungen wie häufiges Kämmen und Bürsten, kann es zu einer unerwünschten Beeinträchtigung der Haarstruktur kommen. Die Beeinträchtigung der Haarstruktur macht sich beispielsweise in einer schlechten Nass- und Trockenkämmbarkeit, einer verstärkten elektrostatischen Aufladung, verstärkter Sprödigkeit, verringerter Höchstreißkraft und Reißdehnung der Haare, Spliss und einem insgesamt verschlechterten äußeren Erscheinungsbild der Haare bemerkbar. Um diesen Nachteilen entgegenzuwirken, ist eine Nachbehandlung mit Konditioniermitteln zwingend notwendig, was wiederum den enormen Zeitaufwand der kompletten Haarbehandlung für den Verbraucher unbefriedigend macht.

Durch die Entwicklung sogenannter 2in1-Shampoos wurde diese Problematik in den letzten Jahren teilweise behoben, denn diese Shampoos umfassen Reinigungs- und Konditioniermittel und machen somit zwei Behandlungsschritte in einem möglich.

Der Nachteil dieser 2in1-Shampoos ist allerdings darin zu sehen, dass das Haar durch eine regelmäßige Behandlung mit den 2in1-Formulierungen häufig ;,überpflegt" ist. Darunter leiden vor allen Dingen Volumen und Body des Haares.
Weiterhin können anschließende Behandlungen wie Coloration oder Dauerwelle beeinträchtigt werden.

Ziel war es daher ein neuartiges Haarbehandlungsmittel zu entwickeln, mit dem eine gleichzeitige Haarreinigung und -pflege möglich ist, ohne dass das Haar dadurch an Attraktivität durch fehlendes Volumen verliert.

Vollkommen überraschend wurde nun festgestellt, dass durch die Kombination einer speziellen Tensidmischung mit ausgewählten Haarpflegestoffen die Nachteile des Standes der Technik in signifikanter Weise behoben werden können.

Gegenstand der Erfindung sind daher kosmetische Haarbehandlungsmittel, enthaltend
a) eine Tensidmischung, die zusammengesetzt ist aus
   (A) mindestens einem anionischen Tensid
   (B) mindestens einem amphoteren Tensid und
   (C) mindestens einem weiteren Tensid, ausgewählt aus der Gruppe der Acylglutamate, der Aminoxide und der Alkylpolyglucoside, sowie
b) mindestens drei weitere Haarpflegestoffe, ausgewählt aus der Gruppe der
   - Fettalkohole,
   - kationischen Tenside,
   - kationischen Polymere,
   - kationisch derivatisierten Proteinhydrolysate,
   - wasserunlöslichen, flüchtigen Silikone und/oder der wasserlöslichen, flüchtigen Silikonen,
   - Vitamine und/oder Provitamine und/oder deren physiologisch verträglichen Derivaten.

Bevorzugte anionische Tenside sind erfindungsgemäß ausgewählt aus der Gruppe der ethoxylierten Alkylsulfattenside, der Alkylsulfate oder der Salze der Ethercarbonsäuren, sowie aus den Gemischen dieser Stoffe. Diese anionischen Tensidklassen sind bevorzugt Salze der Ethercarbonsäuren der Formel R-O-(CH₂-CH₂O)ₓ-CH₂-COOH, in der R eine lineare Alkylgruppe mit 8 bis 30 C-Atomen und X=0 oder 1-16 ist, oder aus Alkylsulfaten oder Alkylpolyglycolethersulfaten der Formel R-(O-CH₂-CH₂)ₓ-OSO₃H, in der R eine bevorzugt lineare Alkylgruppe mit 8 bis 30 C-Atomen und X=0 oder 1 bis 12 ist.

Erfindungsgemäß bevorzugte amphotere Tenside sind solche aus der Gruppe der Alkylbetaine, Amidoalkylbetaine, Amphoacetate oder Amphodiacetate, sowie aus Gemischen dieser Stoffe. Die amphoteren Tensidklassen sind bevorzugt Verbindungen, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂ - C₁₈ - Acylsarcosin.

In einer bevorzugten Ausführungsform der Erfindung wird als dritte Tensidkomponente ein Aminoxid eingesetzt.

Unter Aminoxiden werden erfindungsgemäß Sustanzen der allgemeinen Formel R¹R²R³N-O verstanden, in denen die Substituenten R¹, R² und R³ unabhängig voneinander bevorzugt stehen für Alkylgruppen mit 1 bis 22 C-Atomen oder Alkylamidoalkylgruppen mit 8 bis 25 C-Atomen, mit der Maßgabe, dass mindestens einer dieser Substituenten mindestens 8 C-Atome enthält. Ein erfindungsgemäß bevorzugtes Aminoxid ist das Handelsprodukt Aminoxid WS35, ein N,N-Dimethyl-N(C8-18-kokosacylamidopropyl)amin-N-oxid mit INCl-Bezeichnung Cocamidopropylamine Oxide.

In einer weiteren bevorzugten Ausführungsform der Erfindung wird als dritte Tensidkomponente ein Gemisch aus Acylglutamaten und Aminoxiden oder ein Gemisch aus Acylglutamaten und Alkylpolyglucosiden eingesetzt.

Acylglutamate stellen bekannte anionische Tenside dar, die der Formel (I) folgen, (I), 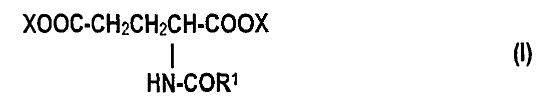 in der R¹CO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht. Ihre Herstellung erfolgt beispielsweise durch Schotten-Baumann Acylierung von Glutaminsäure mit Fettsäuren, Fettsäure-estern oder - chloriden. Verkaufsprodukte sind beispielsweise von der Hoechst AG, Frankfurt/DE oder der Ajinomoto Co. Inc., Tokyo/JP erhältlich. Eine Übersicht zu Herstellung und Eigenschaften der Acyl-glutamate findet sich von M.Takehara et al. in J.Am.Oil.Chem.Soc., 49, 143 (1972). Typische Bei-spiele für geeignete Acylglutamate, die im Sinne der Erfindung in Betracht kommen, sind Aniontenside, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure. Besonders bevorzugt sind Natrium-N-cocoyl- und Natrium-N-stearoyl-L-glutamat.

Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die o entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bevorzugt ist erfindungsgemäß ein Verhältnis der Tensidkomponenten (A) : (B) : (C) von (3 - 6) : 1 : (1 - 2).

Die Haarpflegestoffe b) können erfindungsgemäß sowohl aus einer, als auch aus mehreren der aufgeführten Gruppen ausgewählt sein. Erfindungsgemäß bevorzugt ist es jedoch, wenn mindestens zwei Vertreter aus mindestens zwei Gruppen in den Mitteln enthalten sind.

Als erfindungsgemäße Fettalkohole können eingesetzt werden: gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-,18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Erfindungsgemäß ganz besonders bevorzugte Fettalkohole sind Cetylalkohol Stearylalkohol oder Gemische aus diesen Komponenten.

Die Fettalkohole werden in Mengen von 0,1 - 20 Gew.-%, bezogen auf die gesamte Zubereitung, bevorzugt in Mengen von 0,1-10 Gew.-%, eingesetzt.

Unter kationischen Tensiden im Sinne der Erfindung sind bevorzugt kationische Tenside vom Typ der quartären Ammoniumverbindungen, der Esterquats und der Amidoamine zu verstehen. Bevorzugte quaternäre Ammoniumverbindungen sind Ammoniumhalogenide, insbesondere Chloride und Bromide, wie. Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Unter kationischen Polymeren im Sinne der Erfindung sind beispielsweise kationische Cellulosederivate, wie z.B. eine quaternierte Hydroxyethylcellulose, die unter der Bezeichnung Polymer JR^{®} 400 von Amerchol erhältlich ist, kationische Stärke, Copolymere von Diallylammoniumsalzen und Acrylamiden, quaternierte VinylpyrrolidonNinyl-imidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxypropyl hydrolyzed collagen (Lamequat^{®}L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylamid mit Dimethyldiallyl-ammoniumchlorid (Merquat^{®} 550/Chemviron), Homopolymere des Dimethyldiallyl-ammoniumchlorids (Merquat^{®} 100), Polyaminopolyamide, wie z.B. beschrieben in der FR-A 2252840 sowie deren vernetzte wasserlöslichen Polymere, kationische Chitinderivate, wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol® A-15, Mirapol^{®} AD-1, Mirapol^{®} AZ-1 der Firma Miranol zu verstehen.

Insbesondere bevorzugte kationische Polymere sind kationische Guar-Derivate, kationische Cellulose-Derivate, Homopolymere des Dimethyldiallylammoniumchlorids sowie Copolymere des Dimethyldiallylammoniumchlorids mit Acrylamid.

Ganz besonders bevorzugt im Sinne der Erfindung ist Polyquaternium-10 als kationisches Polymer.

Die kationischen Polymere werden in den erfindungsgemäßen Zusammensetzungen üblicherweise in einer Menge von 0,001 bis 20 Gew.-%, bevorzugt in einer Menge von 0,01 bis 5 Gew.-% und insbesondere in einer Menge von 0,1 bis 2 Gew.-%, jeweils bezogen auf das Gesamtgewicht des Mittels, eingesetzt.

Bei den Proteinhydrolysaten im Sinne der Erfindung handelt es sich üblicherweise um Proteinhydrolysate vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Darüber hinaus können die kationischen Proteinhydrolysate auch noch weiter derivatisiert sein.

Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Ganz besonders bevorzugt sind die kationischen Proteinhydrolysate und -derivate aus Keratin, Collagen, Elastin, Soja, Milch, Weizen, Seide und Mandel, insbesondere bevorzugt ist Hydroxypropyl Hydrolized Wheat Protein, wie es beispielsweise unter dem Handelsnamen Gluadin WQ von der Firma Cognis im Handel erhältlich ist.

In den erfindungsgemäß verwendeten Mitteln sind die Proteinhydrolysate und deren Derivate in Mengen von 0,01 - 10 Gew.-% bezogen auf das gesamte Mittel enthalten. Mengen von 0,1 bis 5 Gew.%, insbesondere 0,1 bis 3 Gew.-%, sind ganz besonders bevorzugt.

Unter wasserunlöslichen Silikonen im Sinne der Erfindung werden solche Silikone verstanden, die bei 20°C zu maximal 0,5 % in Wasser löslich sind.

Unter flüchtigen Silikonen, im Sinne der Erfindung, sind solche zu verstehen, die vollständig verdampfen und keinen Rückstand auf dem Substrat hinterlassen. Bevorzugt sind solche Silikone, von denen eine Probe von 10 g auf einer Petrischale bei 60°C innerhalb von 120 Minuten zu 5-90% verdampft. Besonders bevorzugt sind solche Silikone, die bei diesen Bedingungen zu 10-50% verdampfen.

Erfindungsgemäß bevorzugte Silikonverbindungen sind solche nach Formel (II), 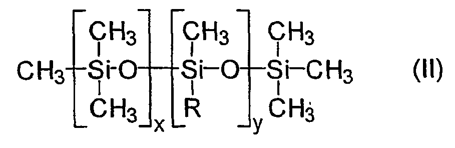 in der x steht für eine Zahl von 1 bis 10, y steht für eine Zahl von 1 bis 10 und R steht für einen Alkylrest mit 2 bis 10 C-Atomen.

Insbesondere bevorzugt sind solche Silikonverbindungen nach Formel (II), in denen die Zahlen x und y für die Zahl 1 stehen.

Insbesondere bevorzugte Silikonkomponenten im Sinne der Erfindung sind die Verbindungen Hexyl Methicone und Caprylyl Methicone, wie sie beispielsweise unter den Handelnamen SilCare 41M10 und SilCare 41M15 von der Firma Clariant im Handel erhältlich sind.

Die Silikonkomponenten werden in den erfindungsgemäßen Mitteln üblicherweise in einer Menge von 0,001 bis 20 Gew.-%, bevorzugt in einer Menge von 0,1 bis 10 Gew.-% und insbesondere in einer Menge von 0,5 bis 2 Gew.-%, jeweils bezogen auf das Gesamtgewicht des Mittels, eingesetzt.

Erfindungsgemäße Vitamine, Pro-Vitamine und Vitaminvorstufen sind üblicherweise aus den Gruppen A, B, C, E, F und H ausgewählt.

Zur Gruppe der als **Vitamin A** bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur **Vitamin B**-Gruppe oder zu dem Vitamin B-Komplex gehören u. a.
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1-5 Gew.-% sind besonders bevorzugt.
Vitamin B₆ (Pyridoxin sowie Pyridoxamin und Pyridoxal).

**Vitamin C** (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

**Vitamin F.** Unter dem Begriff "Vitamin F" werden üblicherweise essentielle Fettsäuren, insbesondere Linolsäure, Linolensäure und Arachidonsäure, verstanden.

**Vitamin H.** Als Vitamin H wird die Verbindung (3a*S*,4*S*,6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Zubereitungen Vitamine, Provitamine und Vitaminvorstufen aus den Gruppen A, B, E und H. Selbstverständlich können auch mehrere Vitamine und Vitaminvorstufen gleichzeitig enthalten sein.

Pantolacton, Pyridoxin und seine Derivate sowie Nicotinsäureamid und Biotin, aber insbesondere Panthenol und seine physiologisch verträglichen Derivate sind besonders bevorzugt.

Es kann erfindungsgemäß bevorzugt sein, zusätzliche weitere Tenside aus der Gruppe der anionischen, kationischen, zwitterionischen, amphoteren oder nichtionischen Tenside einzusetzen.

Als weitere anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (III), 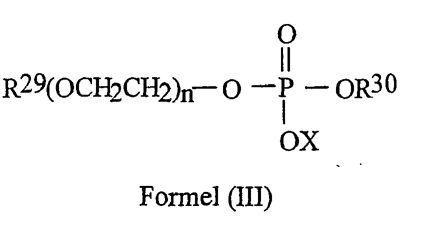
- in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglykolester der Formel (IV)

   R³⁵CO(AlkO)ₙSO₃M Formel (IV)

   in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (V) wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind. 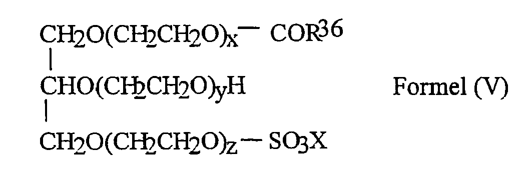 in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (V) eingesetzt, in der R³⁶CO für einen linearen Acylrest mit 8 bis 18 Kohlenstoffatomen steht

Bevorzugte weitere anionische Tenside sind Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Weitere nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (VI)

   R³⁷CO-(OCH₂CHR³⁸)_{w}OR³⁹ Formel (VI),

   in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide.

Als bevorzugte weitere nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden in Mengen von 0,1 - 45 Gew.%, bevorzugt 1-30 Gew.% und ganz besonders bevorzugt von 1-15 Gew.%, bezogen auf das gesamte Mittel, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren bevorzugten Ausführungsform kann die Wirkung des erfindungsgemäßen Mittels durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit, Polyglycerine und Polyglycerinderivate wie beispielsweise Polyglycerinpoly-12-hydroxystearat (Handelsprodukt Dehymuls^{®} PGPH)
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Unter den genannten Emulgatoren-Typen können die Emulgatoren, welche kein Ethylenoxid und/oder Propylenoxid im Molekül enthalten ganz besonders bevorzugt sein.

In einer weiteren bevorzugten Ausführungsform kann die Wirkung des erfindungsgemäßen Mittels durch Ölkörper gesteigert werden. Solche Ölkörper und/oder Fettstoffe sind beispielsweise Fettsäuren, natürliche und synthetische Wachse, welche sowohl in fester Form als auch flüssig in wässriger Dispersion vorliegen können, und natürliche und synthetische kosmetische Ölkomponenten zu verstehen.

Als Fettsäuren können eingesetzt werden lineare und/oder verzweigte, gesättigte und/oder ungesättigte Fettsäuren mit 6-30 Kohlenstoffatomen. Bevorzugt sind Fettsäuren mit 10-22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethythexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1-5 Gew.% sind.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise pflanzliche Öle zu zählen. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-actestether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein,

Erfindungsgemäß geeignet sind aber auch Esteröle. Unter Esterölen sind zu verstehen die Ester von C₆ - C₃₀ - Fettsäuren mit C₂ - C₃₀- Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Erfindungsgemäß geeignet sind auch Dicarbonsäureester. Dicarbonsäureester im Sinne der Erfindung sind Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat

Weiterhin geeignet sind:
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12 oder Cutina^{®} MD.

Erfindungsgemäß ganz besonders bevorzugt sind die Ölkörper aus der Gruppe, die zusammengesetzt ist aus Esterölen von C₆-C₃₀-Fettsäuren mit C₂-C₃₀-Fettalkoholen, Fettsäureglyceride aus Mono-, Di- und Trifettsäureestern von gesättigten und/oder ungesättigten linearen und/oder verzweigten C₆-C₃₀-Fettsäuren mit Glycerin sowie ethoxylierte Fettsäureglyceride aus Mono-, Di- und Trifettsäureestern von gesättigten und/oder ungesättigten linearen und/oder verzweigten C₆-C₃₀-Fettsäuren mit Glycerin. Bevorzugt ist ein Ethoxylierungsgrad von 2 bis 20.

Die Gesamtmenge an Öl- und Fettkomponenten in den erfindungsgemäßen Mitteln beträgt üblicherweise 2-25 Gew.-%, bezogen auf das gesamte Mittel. Mengen von 4-25 Gew.-% sind erfindungsgemäß bevorzugt und mengen von 6 bis 20 Gew.-% sind erfindungsgemäß besonders bevorzugt.

Schließlich können in den erfindungsgemäßen Mitteln Pflanzenextrakte (L) verwendet werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Die Einsatzmenge der Pflanzenextrakte in den erfindungsgemäß verwendeten Mitteln beträgt üblicherweise 0,01 - 50 Gew.%, bezogen auf das gesamte Mittel, bevorzugt 0,1-30 Gew.-%, und insbesondere 0,1-20 Gew.-%.

Vorteilhaft im Sinne der Erfindung können zusätzlich kurzkettige Carbonsäuren (N) eingesetzt werden. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl, C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in α - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 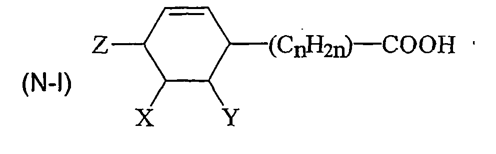 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Ein Herstellungsverfahren ist beispielsweise der US-Patentschrift 3,753,968 zu entnehmen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure.

Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders vorteilhaft hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeignete Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8-22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Weiterhin sind als konditionierende Wirkstoffe geeignet Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie das Handelsprodukt Fancorsil^{®} LIM-1.

Erfindungsgemäß als konditionierende Wirkstoffe ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning^{®} 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80). Ein geeignetes anionisches Silikonöl ist das Produkt Dow Corning^{®}1784.

Die erfindungsgemäßen Haarbehandlungsmittel unterliegen hinsichtlich ihrer Konfektionierungsform keinerlei Beschränkungen und können als Emulsion, Creme, Lösung, Gel, Mousse formuliert werden.

Erfindungsgemäß besonders bevorzugt sind aber Haarbehandlungsmittel, die als Emulsionsshampoo konfektioniert sind.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline,
- sowie Silikonöle, Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol, Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die Monographie von K. H. Schrader, Grundlagen und Rezepturen der Kosmetika, 2. Auflage, Hüthig Buch Verlag Heidelberg, 1989, verwiesen.

Ein zweiter Gegenstand der Erfindung sind kosmetische Haarbehandlungsmittel, enthaltend
a) eine Tensidmischung, die zusammengesetzt ist aus
   (A) mindestens einem anionischen Tensid
   (B) mindestens einem amphoteren Tensid und
   (C) mindestens einem weiteren Tensid, ausgewählt aus der Gruppe der Acylglutamate, der Aminoxide und der Alkylpolyglucoside, sowie
b) vier weitere Haarpflegestoffe, ausgewählt aus je einer Komponente der Gruppen Fettalkohole, kationische Tenside, kationische Polymere, kationisch derivatisierte Proteinhydrolysate, wasserunlösliche oder wasserlösliche flüchtige Silikone und Vitamine und/oder Provitamine und/oder deren physiologisch verträgliche Derivaten.

Bevorzugte weitere Haarpflegestoffe ist insbesondere die Kombination aus Cetylalkohol, Behentrimonium Chloride, Polyquaternium-10 und Caprylyl Methicone.

Ein dritter Gegenstand der Erfindung ist ein Verfahren zur Reinigung und Pflege von Haaren, bei dem ein erfindungsgemäßes Mittel auf die Haare aufgebracht und nach einer Einwirkungszeit wieder ausgespült wird.

Ein vierter Gegenstand der Erfindung ist die Verwendung der erfindungsgemäßen Mittel zur gleichzeitigen Reinigung und Konditionierung von Haaren.

Die folgenden Beispiele sollen den Gegenstand der Erfindung näher erläutern, ohne ihn darauf zu beschränken - alle Gewichtsangaben beziehen sich, sofern nicht anders angegeben, auf Gew.%.

### Beispiele

| | A | B | C |
|---|---|---|---|
| | Gew.% | Gew.% | Gew.% |
| Sodium Laureth Sulfate | 10 | 12 | 9 |
| Cocamidopropyl Betaine | 4 | 2 | 6 |
| Disodium Cocoyl Glutamate | 1 | 1 | 2 |
| Cocamidopropyl Aminoxide | 1 | | 2 |
| Coco-Glucoside | | 2 | |
| Behentrimonium Chloride | 1 | 1,5 | 1 |
| Cetyl Alcohol | 0,3 | | 0,3 |
| Stearyl Alcohol | | 0,7 | 0,3 |
| Polyquaternium-10 | 0,1 | 0,2 | 0,1 |
| Glycol Distearate | | | 1 |
| Caprylyl Methicone | 0,5 | | 0,4 |
| Laurdimonium Hydroxypropyl Hydrolized Wheat Protein | | 0,5 | |
| Panthenol | | | 0,3 |
| Konservierungsmittel | q.s. | q.s. | q.s. |
| Parfüm | q.s. | q.s. | q.s. |
| Wasser | ad 100 | ad 100 | ad 100 |

Das erfindungsgemäße Mittel zeigt in Haarstudiountersuchungen - d.h. Anwendung des Produktes im sogenannten Halbseitentest unter Verwendung von 2 g Produkt für die Vorwäsche und 3 g Produkt für die Hauptwäsche und anschließender friseurtechnischer Beurteilung - signifikante Vorteile in den Parametern Naßkämmbarkeit, Griff, Volumen, Belastung/Beschwerung im Vergleich zu marktüblichen Produkten von Wettbewerbern.

Weiterhin wurde die gute Nasskämmbarkeit des erfindungsgemäßen Shampoos im Vergleich zu marktüblichen 2in1-Shampoos über biophysikalische Messungen gezeigt (Meßprinzip: Messung der erforderlichen Kraft zum Durchkämmen von Haarsträhnen. Strähnen Alkinco 6634 mediumblondiert, Messung von 20 Strähnen je Probe und Mittelwertbildung)

| Produkt | eduktion Nasskämmarbeit in % |
|---|---|
| Erfindungsgemässes Shampoo | 56,6 |
| Wettbewerber P (2in1 Shampoo) | 44,5 |
| Wettbewerber GF (2in1 Shampoo) | 32,0 |
| Wettbewerber WG (2in1 Shampoo) | 42,9 |
| Wettbewerber HE (2in1 Shampoo) | 20,7 |

Die Anti-Spliss Wirkung wurde mit folgendem Verfahren ermittelt:

### Vorbehandlung der Haarsträhnen:

Die Haarsträhnen wurden mit einer 10%igen alkalischen Sodium Laureth Sulfate - Lösung 15 Minuten in einem Ultraschallbad vorgereinigt

### Produktapplikation :

Je Produkt wurden 9 Haarsträhnen behandelt, zuzüglich einer Kontrollmessreihe. Hierzu wurden die Haarsträhnen mit Leitungswasser angefeuchtet und jeweils 1 g Produkt pro g Haarsträhne eingearbeitet. Nach einer 5-minütigen Einwirkzeit wurde das Haar 90 Sekunden unter kaltem, fließendem Leitungswasser abgespült. Die Vorkonditionierung der Haare erfolgte bei 25°C und einer relativen Luftfeuchtigkeit von 40% über mindestens 12 Stunden.

### Versuchsmethode:

Die Haarsträhnen wurden in einer klimatisierten Kammer (25°C, 40% relative Luftfeuchtigkeit) 20000 mal gekämmt. Mittels eines Feinstsiebes (Maschenweite 200 µm) wurden hiernach die geschädigten von den ungeschädigten Haaren getrennt und jeweils ausgewogen.

Ergebnis im Vergleich zu marktüblichen 2in1 Shampoos:

| Produkt | Spliss-Reduktion bez. auf Nullwert in % |
|---|---|
| Erfindungsgemässes Shampoo | 88,0 |
| Wettbewerber GF (2in1 Shampoo) | 49,7 |
| Wettbewerber LO (2in1 Shampoo) | 55,6 |
| Wettbewerber JLD (3in1 Shampoo) | 21,8 |

## Patentansprüche

1. Kosmetisches Haarbehandlungsmittel, enthaltend
a) eine Tensidmischung, die zusammengesetzt ist aus
(A) mindestens einem anionischen Tensid
(B) mindestens einem amphoteren Tensid und
(C) mindestens einem weiteren Tensid, ausgewählt aus der Gruppe der Acylglutamate, der Aminoxide und der Alkylpolyglucoside, sowie
b) mindestens drei weitere Haarpflegestoffe, ausgewählt aus der Gruppe der
- Fettalkohole,
- kationischen Tenside,
- kationischen Polymere,
- kationisch derivatisierten Proteinhydrolysate,
- wasserunlöslichen, flüchtigen Silikone und/oder der wasserlöslichen, flüchtigen Silikone,
- Vitamine und/oder Provitamine und/oder deren physiologisch verträglichen Derivaten.

2. Kosmetisches Haarbehandlungsmittel nach Anspruch 1, **dadurch gekennzeichnet, dass** das anionische Tensid ausgewählt ist aus der Gruppe der ethoxylierten Alkylsulfate, der Alkylsulfate oder der Salze der Ethercarbonsäuren, sowie aus Gemischen dieser Stoffe.

3. Kosmetisches Mittel nach einem der Ansprüche 1 oder 2, **dadurch gekennzeichnet, dass** das amphotere Tensid ausgewählt ist aus der Gruppe der Alkylbetaine, Amidoalkylbetaine, Amphoacetate oder Amphodiacetate, sowie aus Gemischen dieser Stoffe.

4. Kosmetisches Mittel nach einem der Ansprüche 1 bis 3, **dadurch gekennzeichnet, dass** die dritte Tensidkomponente ein Aminoxid ist.

5. Kosmetisches Mittel nach einem der Ansprüche 1 bis 3, **dadurch gekennzeichnet, dass** die dritte Tensidkomponente ein Gemisch aus Acylglutamaten und Aminoxiden oder ein Gemisch aus Acylglutamaten und Alkylpolyglucosiden ist.

6. Kosmetisches Mittel nach einem der Ansprüche 1 bis 5, **dadurch gekennzeichnet, dass** das Verhältnis der Tensidkomponenten (A) : (B) : (C) (3 - 6) :1: (1-2) beträgt.

7. Kosmetisches Haarbehandlungsmittel gemäß Anspruch 1, enthaltend
a) eine Tensidmischung, die zusammengesetzt ist aus
(A) mindestens einem anionischen Tensid
(B) mindestens einem amphoteren Tensid und
(C) mindestens einem weiteren Tensid, ausgewählt aus der Gruppe der Acylglutamate, der Aminoxide und der Alkylpolyglucoside, sowie
b) vier weitere Haarpflegestoffe, ausgewählt aus je einer Komponente der Gruppen Fettalkohole, kationische Tenside, kationische Polymere, kationisch derivatisierte Proteinhydrolysate, wasserunlösliche oder wasserlösliche flüchtige Silikone und Vitamine und/oder Provitamine und/oder deren physiologisch verträgliche Derivaten.

8. Kosmetisches Haarbehandlungsmittel nach Anspruch 7, **dadurch** gekannzeichnet, dass als Haarpflegestoffe b) Cetylalkohol, Behentrimonium Chloride, Polyquaternium-10 und Caprylyl Methicone eingesetzt werden.

9. Verfahren zur Reinigung und Pflege von Haaren, **dadurch gekennzeichnet, dass** ein Mittel, nach einem der Ansprüche 1 bis 8 auf die Haare aufgebracht wird und nach einen Einwirkungszeit wieder ausgespült wird.

10. Verwendung eines Mittels nach einem der Ansprüche 1 bis 8 als 2in1-Shampoo zur gleichzeitigen Reinigung und Konditionierung von Haaren.

## Claims

1. Cosmetic hair treatment agent comprising
a) a surfactant mixture which is composed of
(A) at least one anionic surfactant
(B) at least one amphoteric surfactant and
(C) at least one further surfactant selected from the group of acyl glutamates, amine oxides and alkyl polyglucosides, and
b) at least three further haircare substances selected from the group of
- fatty alcohols,
- cationic surfactants,
- cationic polymers,
- cationically derivatized protein hydrolysates,
- water-insoluble, volatile silicones and/or water-soluble, volatile silicones,
- vitamins and/or provitamins and/or physiologically compatible derivatives thereof.

2. Cosmetic hair treatment agent according to Claim 1, **characterized in that** the anionic surfactant is selected from the group of ethoxylated alkyl sulphates, alkyl sulphates or the salts of ether carboxylic acids, and also from mixtures of these substances.

3. Cosmetic agent according to one of Claims 1 or 2, **characterized in that** the amphoteric surfactant is selected from the group of alkylbetaines, amido-alkylbetaines, amphoacetates or amphodiacetates, and from mixtures of these substances.

4. Cosmetic agent according to one of Claims 1 to 3, **characterized in that** the third surfactant component is an amine oxide.

5. Cosmetic agent according to one of Claims 1 to 3, **characterized in that** the third surfactant component is a mixture of acyl glutamates and amine oxides or a mixture of acyl glutamates and alkyl polyglucosides.

6. Cosmetic agent according to one of Claims 1 to 5, **characterized in that** the ratio of the surfactant components (A):(B):(C) is (3-6):1:(1-2).

7. Cosmetic hair treatment agent according to Claim 1 comprising
a) a surfactant mixture which is composed of
(A) at least one anionic surfactant
(B) at least one amphoteric surfactant and
(C) at least one further surfactant selected from the group of acyl glutamates, amine oxides and alkyl polyglucosides, and
b) four further haircare substances selected from one component from each of the groups of fatty alcohols, cationic surfactants, cationic polymers, cationically derivatized protein hydrolysates, water-insoluble or water-soluble volatile silicones and vitamins and/or provitamins and/or physiologically compatible derivatives thereof.

8. Cosmetic hair treatment agent according to Claim 7, **characterized in that** cetyl alcohol, behentrimonium chloride, polyquaternium-10 and caprylylmethicone are used as haircare substances b).

9. Method for the cleansing and care of hair, **characterized in that** an agent according to one of Claims 1 to 8 is applied to the hair and, after a contact time, is rinsed out again.

10. The use of an agent according to one of Claims 1 to 8 as 2 in 1 shampoo for the simultaneous

## Revendications

1. Agent cosmétique de traitement capillaire, contenant
a) un mélange de tensioactifs constitué par
(A) au moins un tensioactif anionique,
(B) au moins un tensioactif amphotère, et
(C) au moins un autre tensioactif, choisi dans le groupe constitué par les acylglutamates, les oxydes d'amine et les alkylpolyglucosides, ainsi que
b) au moins trois autres agents de soin capillaire, choisi dans le groupe formé par
- des alcools gras,
- des tensioactifs cationiques,
- des polymères cationiques,
- des hydrolysats de protéines cationiques dérivatisés,
- des silicones volatils insolubles dans l'eau et/ou des silicones volatils solubles dans l'eau,
- des vitamines et/ou des provitamines et/ou leurs dérivés physiologiquement compatibles.

2. Agent cosmétique de traitement capillaire selon la revendication 1, **caractérisé en ce que** le tensioactif anionique est choisi dans le groupe constitué par les alkylsulfates éthoxylés, les alkylsulfates ou les sels d'acides éthercarboxyliques, ainsi que les mélanges de ces composés.

3. Agent cosmétique selon l'une des revendications 1 ou 2, **caractérisé en ce que** le tensioactif amphotère est choisi dans le groupe constitué par les alkylbétaines, les amidoalkylbétaines, les amphoacétates ou amphodiacétates, ainsi que les mélanges de ces composés.

4. Agent cosmétique selon l'une des revendications 1 à 3, **caractérisé en ce que** le troisième composant tensioactif est un oxyde d'amine.

5. Agent cosmétique selon l'une des revendications 1 à 3, **caractérisé en ce que** le troisième composant tensioactif est un mélange d'acylglutamates et d'oxydes d'amine ou un mélange d'acylglutamates et d'alkylpolyglucosides.

6. Agent cosmétique selon l'une des revendications 1 à 5, **caractérisé en ce que** le rapport entre les composants tensioactifs (A):(B):(C) est de (3-6):1:(1 - 2).

7. Agent cosmétique de traitement capillaire selon la revendication 1, contenant
a) un mélange de tensioactifs constitué par
(A) au moins un tensioactif anionique,
(B) au moins un tensioactif amphotère, et
(C) au moins un autre tensioactif, choisi dans le groupe constitué par les acylglutamates, les oxydes d'amine et les alkylpolyglucosides, ainsi que
b) quatre autres agents de soin capillaire, choisis chacun parmi un des composants du groupe formé par les alcools gras, les tensioactifs cationiques, les polymères cationiques, les hydrolysats de protéines cationiques dérivatisés, les silicones volatils solubles ou insolubles dans l'eau, et des vitamines et/ou provitamines et/ou leurs dérivés physiologiquement compatibles.

8. Agent cosmétique de traitement capillaire selon la revendication 7, **caractérisé en ce que** comme agent de soin capillaire, on utilise b) l'alcool cétylique, le chlorure de trimonium béhénique, le polyquaternium-10 et le Caprylyl Methicone.

9. Procédé de lavage et de soin capillaire, **caractérisé en ce que** l'on applique sur les cheveux un agent selon l'une quelconque des revendications 1 à 8 et après un temps d'action, on l'élimine par rinçage.

10. Utilisation d'un agent selon l'une quelconque des revendications 1 à 8 en tant que shampooing « 2 en 1 » pour le lavage et le conditionnement simultané des cheveux.
